# EUROPEAN PATENT APPLICATION

(11) **EP 4 302 710 A1**
(43) Date of publication of application: **10.01.2024**
(21) Application number: 22811272.8
(22) Date of filing: 23.05.2022
(51) Int. Cl.: A61B 17/28, A61B 17/94

(54) **ENDOSCOPIC TREATMENT IMPLEMENT**

(30) Priority: 25.05.2021 JP 2021087785
(71) Applicant: Homma, Kiyoaki, Sakata-shi Yamagata 998-0061 (JP)
(72) Inventor: Homma, Kiyoaki, Sakata-shi Yamagata 998-0061 (JP)
(74) Representative: Schweiger, Martin
(86) International application number: PCT/JP2022/021059
(87) International publication number: WO 2022/250002

(57) **Abstract**

A treatment instrument for endoscopes includes a sheath, an operation wire, an operation unit, a treatment unit support member having a tube part and a pair of arm parts, treatment pieces, a pair of driving links, and a forward and backward movement transmission rod. The treatment unit support member forms treatment liquid supply paths between the arm parts and the treatment pieces by two concave surface parts formed on inner surface of the arm parts. An inner concave wall having a through hole passing through and guiding the forward and backward movement transmission rod is formed in the tube part. The concave surface parts form the treatment liquid supply path by a pair of liquid supply parts in the through hole. An opening and closing degree of the treatment piece is restricted by a stopper of the arm part and a stopper of the tube part.

## Description

### TECHNICAL FIELD

The present invention relates to a treatment instrument for endoscopes which is inserted into and removed from a channel of an endoscope, and has a treatment unit in a distal end, the treatment unit performing treatment of a lesion area in a tissue from a living body.

### BACKGROUND ART

The endoscope is used for a treatment instrument for endoscopes which is inserted into a body cavity and performs a direct examination and a medical treatment of a lesion area in a tissue from a living body according to a minimal invasive. The treatment instrument for endoscope has a treatment instrument in a distal end, the treatment instrument being accommodated in a channel (a long hole) of the endoscope to be insertable and removable and provided for a treatment of the lesion area (removal of an affected area in the tissue from the living body, sample collection, resection and hemostasis) in a visual field of the endoscope. The endoscope is generally operated by a doctor, and the treatment instrument is generally operated by the other doctor or technologist.

Terms used in the description relating to a treatment instrument for endoscopes of the following patent literature 1 are terms used in the same patent literature.

The treatment instrument for endoscopes shown in the patent literature 1 is provided with an operation unit having an operation member which is disposed within a sheath to be movable forward and backward, the sheath being constructed by covering a resin sheath with a coil sheath, and a liquid injection port which is disposed in a proximal end part of the coil sheath and injects a treatment liquid to the coil sheath, and operating the operating member to move forward and backward, a distal end cover which is connected to a distal end part of the coil sheath, a treatment unit support member, and a pair of forceps pieces. The pair of forceps pieces construct a treatment unit.

The treatment unit support member has a flow path forming member which is accommodated in a cylindrical space part having a flange like part disposed in a distal end cover and an abutting part, and has a center hole into which a distal end part of the operation member is inserted, and a pair of brackets which extend to a front side of the distal end cover from opposite positions in the distal end of the flow path forming member.

In the pair of forceps pieces, intermediate cross parts are supported to a pair of brackets by a swing shaft, rear portions of the intermediate cross parts form a rhombic link part, and a pair of links in a front side of the rhombic link part are integrally formed with the respective pair of forceps pieces. A pair of links in a rear side of the rhombic link parts are connected to the distal end part of the operation member in an outer side of the distal end cover. Thus, in the treatment instrument, the rhombic link part opens and closes in response to a forward and backward movement of the operation member. Therefore, the treatment unit constructed by a pair of forceps pieces in a front side portion of the intermediate cross part opens and closes, thereby performing treatment of an affected area in the living body.

Further, in the treatment instrument for endoscopes, when the operation member is pulled rearward and a pair of forceps pieces are in a closed state, the flow path forming member moves backward to come into contact with the abutting part, and a passage in an outer peripheral side of the flow path forming member is closed. Then, the treatment instrument for endoscopes allows a treatment liquid supplied within the coil sheath to flow out to the distal end side of a pair of forceps pieces through a first liquid supply part which corresponds to a gap between a center hole of the flow path forming member and the operation member inserted into the center hole, and further through a second liquid supply part in the distal end part of the distal end cover.

Further, in the treatment instrument for endoscopes, when the operation member is moved forward and a pair of forceps pieces are in an open state, a large-diameter part disposed in the distal end side of the operation member moves forward to close the first liquid supply part, and the flow path forming member moves forward and moves away from the abutting part to open the gap in the outer peripheral side of the flow path forming member 7. Then, the treatment instrument for endoscopes allows the treatment liquid supplied within the coil sheath to flow out to the distal end side of a pair of forceps pieces through the gap and further through the second liquid supply part.

### CITATION LIST

### PATENT LITERATURE

Patent Literature 1: WO2017/134757

### SUMMARY OF INVENTION

### TECHNICAL PROBLEM

The treatment instrument for endoscopes shown in the patent literature 1 allows the treatment liquid supplied within the coil sheath to flow out to the distal end side of a pair of forceps pieces in both of the closed state and the open state of a pair of forceps pieces. In the treatment instrument for endoscopes, the flow of the treatment liquid flowing out of the second liquid supply part is greatly widened and a flow velocity becomes slow because of the following reasons (1), (2) and (3). In the reason (1), a liquid supply pressure of the treatment liquid supplied within the coil sheath is released in the distal end of the distal end cover. In the reason (2), a distance from the distal end of the distal end cover to the distal end of a pair of forceps pieces is long. In the reason (3), the liquid flow is throttled by an inner portion of the distal end cover and the liquid flows in a curved manner. Because of the slow flow velocity, the conventional treatment instrument for endoscopes can not effectively clean a bloodshed, a stagnation and a turbidity of a treated area in the living body, and has a problem that the conventional treatment instrument for endoscopes does not sufficiently produce an effect on a cauterization incision.

The present invention is made for solving the problem mentioned above, and an object of the present invention is to provide a treatment instrument for endoscopes which can accurately supply liquid having a sufficiently great flow velocity along a pair of treatment pieces (treatment units) or each of the treatment pieces in any of a closed state and an open state of a pair of treatment pieces, and can create two liquid flows energetically flowing along each of the treatment pieces, and one liquid flow energetically flowing toward a center of an intermediate part in an open state of a pair of treatment pieces when a pair of treatment pieces are in an open state, thereby effectively cleaning up the bloodshed, the stagnation and the turbidity in the treated area of the living body and producing a sufficient effect on the cauterization incision.

Further, an object of the present invention is to provide a treatment instrument for endoscopes which can restrict an opening and closing angle of a treatment piece in which a distal end is opened and closed by a link mechanism.

### SOLUTION TO PROBLEM

In order to achieve the object mentioned above, a first aspect of the present invention is provided with a sheath which is inserted into and removed from an endoscope channel and has a flexibility, an operation wire which is disposed within the sheath to be movable forward and backward, an operation unit which is disposed in a proximal end part of the sheath, has a liquid injection port injecting a treatment liquid into the sheath and operates the operation wire, a treatment unit support member having a tube part which is connected to a distal end part of the sheath and a pair of arm parts which extend to a front side from opposite positions to the distal end of the tube part, a link mechanism constructed by a pair of opening and closing links which are rotatably connected one ends thereof by a first connection shaft in crossing parts intersecting in an overlapping manner, and a pair of driving links which are rotatably connected one ends thereof to the other ends of the pair of opening and closing links respectively by a second connection shaft and a third connection shaft in an overlapping manner, and are rotatably connected the other ends thereof by a fourth connection shaft in an overlapping manner, in which side surfaces of the driving links and/or the opening and closing links overlapping both side edge parts of the pair of arm parts of the treatment unit support member are in contact while rubbing, and a position of the first connection shaft is fixed to the arm part, a pair of treatment pieces which are adapted to extend in a distal end direction from the one ends of the pair of opening and closing links of the link mechanism, and form a treatment unit performing treatment of an affected area in a living body by a change between a closed state and an open state of the distal end side, and a forward and backward movement transmission rod which moves the fourth connection shaft forward and backward by a forward and backward action force of the operation wire,
wherein in the treatment unit support member, a concave surface part is formed in an inner surface of the pair of arm parts, the concave surface part being formed into a concave shape in a cross section which is vertical to a longitudinal direction,
wherein an inner concave wall having a through hole inserting and guiding the forward and backward movement transmission rod is formed in the tube part, and the through hole has a liquid supply part allowing the treatment liquid supplied through an inner side of the sheath to flow out to the pair of concave surface parts, and
wherein the arm part has a treatment liquid supply path which jets the treatment liquid flowed out of the pair of concave surface parts, in a gap between the concave surface part formed in the inner surface and a side surface of the link mechanism.

Further, a second aspect of the present invention is the treatment instrument for endoscopes according to the first aspect, wherein the treatment liquid supply path is formed in such a manner that the treatment liquid is wider than a diameter of the first connection shaft and the treatment liquid flows straightly in both sides of the first connection shaft.

Further, a third aspect of the present invention is the treatment instrument for endoscopes according to the second aspect, wherein the concave surface part is a concave surface which is formed in each of opposite surfaces of the pair of arm parts in such a manner that a center of curvature conforms to an extended center line of the operation wire, and a planar side surface part in an outer side of the crossing part of the opening and closing link is in slide contact with both side edge parts via the concave surface part in the opposite surfaces of the arm part in the same side.

Further, a fourth aspect of the present invention is the treatment instrument for endoscopes according to any one of the first to third aspects, wherein the arm part is provided with a stopper which is disposed at a position preventing movement of the driving link and/or the opening and closing link caused by the forward and backward action of the forward and backward movement transmission rod.

Further, a fifth aspect of the present invention is the treatment instrument for endoscopes according to any one of the first to third aspects, wherein the forward and backward movement transmission rod is provided with a distal end part having a greater diameter than a shorter diameter of the liquid supply part and formed into a fist shape, in a distal end of the forward and backward movement transmission rod passing through the cylinder tube part.

Further, a sixth aspect of the present invention is the treatment instrument for endoscopes according to any one of the first to fifth aspects, wherein the liquid supply part is formed into an oval shape which is longer in the facing direction of the pair of arm parts.

### EFFECT OF INVENTION

According to the present invention, it is possible to accurately jet the liquid having the sufficiently great flow velocity along a pair of treatment pieces or each of the treatment pieces in any of the closed state and the open state of a pair of treatment pieces, and create two liquid flows energetically flowing along each of the treatment pieces and one liquid flow energetically flowing toward the center of the intermediate part when a pair of treatment pieces are in the open state. Therefore, according to the present invention, it is possible to provide the treatment instrument for endoscopes which can effectively clean and remove the bloodshed, the stagnation and the turbidity in the treated area of the living body, and can achieve the sufficient effect for the cauterization incision.

Further, according to the present invention, the stopper disposed in the arm part is arranged at the position preventing the movement caused by the forward and backward action of the forward and backward movement transmission rod or the movement of the link. Therefore, it is possible to restrict the angle at which the treatment piece opens.

### BRIEF DESCRIPTION OF DRAWINGS

Fig. 1 is a view for describing an endoscope which includes a treatment instrument for endoscopes according to an embodiment 1 of the present invention.
Fig. 2 is a view showing a whole of the treatment instrument for endoscopes according to the embodiment 1 of the present invention.
Fig. 3 is a view for describing an outer appearance of a front face of the treatment instrument for endoscopes according to the embodiment 1 of the present invention, in which Fig. 3A is a front elevational outer appearance view which shows a state that a pair of treatment pieces are open and in which a sheath is omitted, and Fig. 3B is a front elevational outer appearance view which shows a state that a pair of treatment pieces are closed and in which the sheath is omitted.

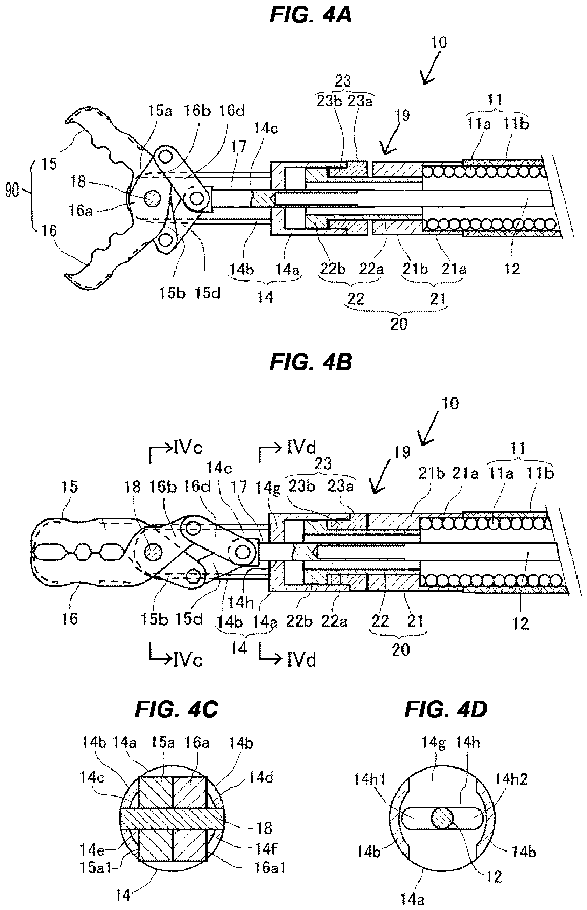

Fig. 4 is a view for describing a cross section of the front face of the treatment instrument for endoscopes according to the embodiment 1 of the present invention, in which Fig. 4A is a front elevational cross sectional view of a distal end portion of the treatment instrument in a state that the treatment pieces are open, Fig. 4B is a front elevational cross sectional view of the distal end portion of the treatment instrument in a state that the treatment pieces are closed, Fig. 4C is a cross sectional view along a line IVc-IVc in Fig. 4B, and Fig. 4D is a cross sectional view along a line IVd-IVd in Fig. 4B.
Fig. 5 is a view showing a cross section of a side face of the treatment instrument for endoscopes according to the embodiment 1 of the present invention, and showing a cross section along a line V-V in Fig. 3B.

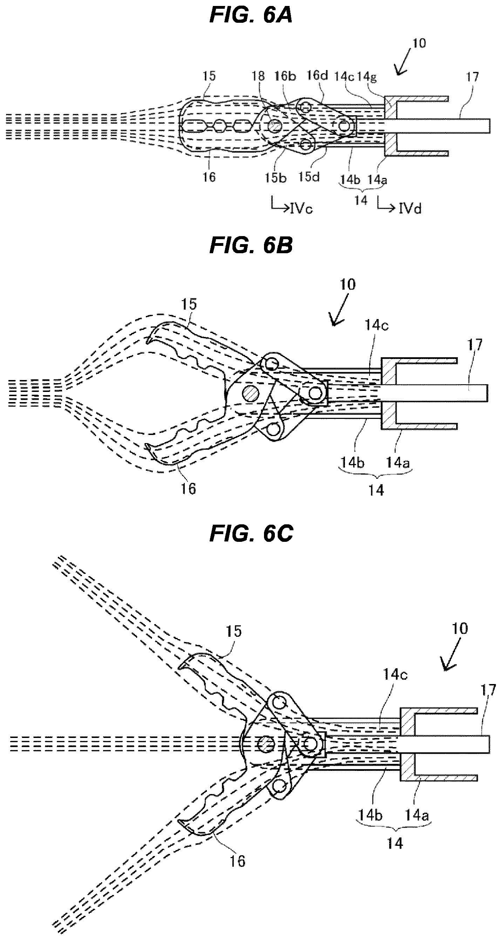

Fig. 6 is a view for describing a flow of a treatment liquid which is jetted from the treatment instrument for endoscopes according to the embodiment 1 of the present invention, in which Fig. 6A is a view showing a state that a liquid circulating in an inner part of a treatment support member flows out of a distal end of the treatment unit support member when the treatment pieces are in the closed state, Fig. 6B is a view showing a state that the liquid circulating in the inner part of the treatment unit support member flows out of the distal end of the treatment unit support member when the treatment pieces are in an intermediate opening degree, and Fig. 6C is a view showing a state that the liquid circulating in the inner part of the treatment unit support member flows out of the distal end of the treatment unit support member when the treatment pieces are in the maximum opening degree.
Fig. 7 is a view for describing a cross section of a front face of a treatment instrument for endoscopes according to an embodiment 2 of the present invention.
Fig. 8 is a view for describing a treatment unit support member of a treatment instrument for endoscopes according to an embodiment 3 of the present invention, in which Fig. 8A is an overall perspective view, Fig. 8B is a right side elevational view, Fig. 8C is a left side elevational view, Fig. 8D is a top elevational view, Fig. 8E is an enlarged view of a portion A in Fig. 8A, and Fig. 8F is an enlarged view of a portion B in Fig. 8A.
Fig. 9 is a cross sectional view for describing a configuration and a motion of a link mechanism of the treatment instrument for endoscopes according to the embodiment 3 of the present invention.
Fig. 10 is a perspective view showing an outer appearance of the link mechanism of the treatment instrument for endoscopes according to the embodiment 3 of the present invention.
Fig. 11 is a view for describing a principle of the link mechanism of the treatment instrument for endoscopes according to the embodiment 3 of the present invention.

### DESCRIPTION OF EMBODIMENTS

A description will be given below of embodiments according to a treatment instrument for endoscopes according to the present invention with reference to the accompanying drawings. In the following description, a side where a treatment unit is positioned is called as a distal end side, and a side where an operation unit is positioned is called as a proximal end side.

### [Embodiment 1]

### [Endoscope]

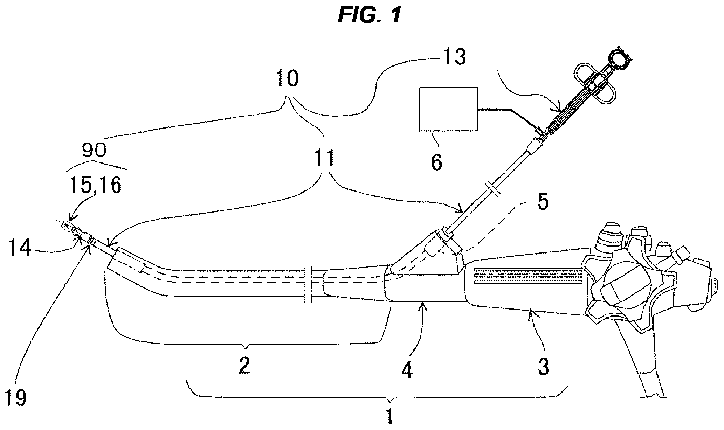

Fig. 1 is a view showing an endoscope 1 to which a treatment instrument for endoscopes according to an embodiment 1 is applied. The endoscope 1 is provided with an elongated insertion unit 2 for inserting into a body cavity of a living body, an endoscope operation unit 3 which is disposed in a proximal end side and has a dial for operating in a curved manner a distal end of the insertion unit 2 in vertical and horizontal directions, and a treatment instrument introducing unit 4 which is arranged in such a manner as to connect between the insertion unit 2 and the endoscope operation unit 3, and to which an insertion port inserting a treatment instrument for endoscopes 10 is opened. The endoscope 1 is structured such that a tubular endoscope channel 5 formed in a longitudinal direction from the treatment instrument introducing unit 4 toward a distal end of the insertion unit 2 is opened, and a sheath 11 is inserted into the endoscope channel 5. A pair of treatment pieces 15 and 16 mentioned later of the treatment instrument for endoscopes 10 perform treatment of an affected area in the living body and are attached to a distal end side of the sheath 11.

### [Basic Structure of Treatment Instrument for Endoscopes]

The treatment instrument for endoscopes 10 according to the embodiment 1 is provided with a treatment unit constructed by a pair of treatment pieces 15 and 16 corresponding to a cup-shaped forceps which pinches a treated part (an affected area) of a tissue from a living body within the body cavity and performs an electrical cauterization. The treatment instrument for endoscopes 10 feeds a treatment liquid through a sheath 11 by a liquid supply means 6, and jets the treatment liquid ahead of a pair of treatment pieces 15 and 16, so that the treatment instrument for endoscopes 10 can clean the treated part (the affected area) of the tissue from the living body within the body cavity. A pair of treatment pieces 15 and 16 are not limited to the electrical cauterization cup-shaped forceps.

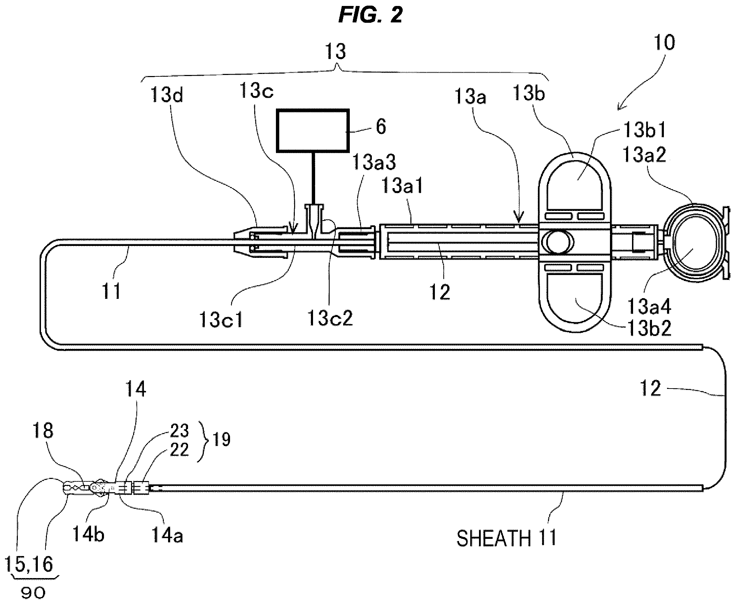

As shown in Figs. 2, 3A, 3B, 4A and 4B, the treatment instrument for endoscopes 10 according to the present embodiment is provided with an elongated flexible tubular sheath 11, an operation wire 12 which passes through the sheath 11, an operation unit 13 which is connected to a proximal end side of the operation wire 12, a treatment unit 90 which is connected to a distal end side of the operation wire 12 via a forward and backward movement transmission rod 17 and a link mechanism, and a treatment unit support member 14 which is attached to a distal end side of the sheath 11 via a rotatable connection part 19 and supports the treatment unit 90. To the rotatable connection part 19, a base end side connecting tubular part 20 and a distal end side connecting tubular part 23 are rotatably connected.

The treatment unit 90 is constructed by a pair of treatment pieces 15 and 16 which are opened and closed by a pair of driving links 15d and 16d and opening and closing links 15b and 16b, and is adapted to open and close the treatment pieces 15 and 16 via the link mechanisms on the basis of a forward and backward action of the operation wire 12 via the forward and backward movement transmission rod 17.

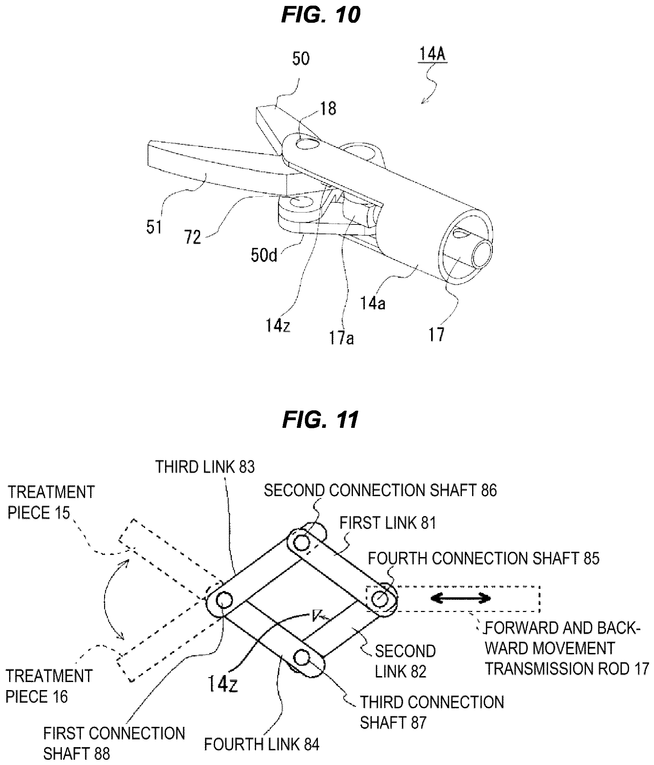

A description will be given of a principle and a structure of the link mechanism applied to the present embodiment with reference to Fig. 11. The link mechanism according to the present embodiment is a pantograph type four-joint link mechanism as shown in Fig. 11, is provided with a first link 81 and a second link 82 (called as "a pair of driving links") which are rotatably connected in one ends thereof in an overlapping manner by a fourth connection shaft 85 connected to the forward and backward movement transmission rod 17 performing a liner motion, and a third link 83 and a fourth link 84 (called as "a pair of opening and closing links") which are rotatably connected in one ends thereof in an overlapping manner to the other ends of the first link 81 and the second link 82 by a second connection shaft 86 and a third connection shaft 87, and are rotatably in the other ends thereof in an overlapping manner by a first connection shaft 88 a position thereof being fixed. An overlapping width of the first to fourth links 81 to 84 is set to be approximately the same as a distance between a pair of arm parts of the treatment unit support member, the fourth connection shaft 85 comes to a point of effort for a pair of driving links and the first connection shaft 88 comes to a point of support of a pair of opening and closing links in correspondence to the forward and backward movement of the forward and backward movement transmission rod 17, and the treatment piece 16 extending from the other end of the third link 83, and the treatment piece 15 extending from the other end of the fourth link 84 are adapted to open and close, the third link 83 and the fourth link 84 corresponding to a pair of opening and closing links. Reference symbol 14z in Fig. 11 denotes a stopper mentioned later.

As mentioned above, the link mechanism employed for the present embodiment is constructed by a pair of opening and closing links (the third link 83 and the fourth link 84) which are formed by extending the treatment pieces 15 and 16 from one ends thereof and are rotatably connected by the first connection shaft 88, and a pair of driving links (the first link 81 and the second link 82) which are connected in one ends thereof to the other ends of the pair of opening and closing links by the second connection shaft 86 and the third connection shaft 87, and are rotatably connected in the other ends thereof by the fourth connection shaft 85. The fourth connection shaft 85 of the driving link is provided with the forward and backward movement transmission rod 17 which are moved forward and backward by the application of the forward and backward action force of the operation wire. The link mechanism is adapted to open and close a pair of treatment pieces 15 and 16 extended from the opening and closing link, by moving forward and backward the forward and backward movement transmission rod 17.

The treatment instrument for endoscopes 10 can be smoothly introduced into the body cavity with no scratch from the treatment pieces 15 and 16 to the sheath 11 since a hydrophilic coating film is formed on an outer surface of at least a portion of the sheath 11 which is inserted into the body cavity, and on an outer surface of the treatment unit support member 14.

In the sheath 11, a resin outer cover 11b is coated on a coil sheath 11a which is closely attached and long coiled, and the sheath 11 has a flexibility for being inserted into and removed from the endoscope channel 5.

The sheath 11 is an elongated tubular body which has a length, for example, between 500 and 2600 mm, has a flexibility and has an appropriate stiffness (flexure resistance). The sheath 11 according to the present embodiment is constructed by the coil sheath 11a, and the resin outer cover 11b which is covered on the outer surface of the coil sheath 11a. The coil sheath 11a preferably employs a coil sheath which has a rectangular cross sectional shape and coiled by closely attaching a metal material such as a stainless wire, for example. The resin outer cover 11b is made of PTFE, PEEK, PPS, polyethylene or polyimide which have a flexibility and an electric insulation property. The hydrophilic coating film is formed on an electric insulating coating film in an overlapping manner.

In the sheath 11, the electric insulating coating film is formed on an inner surface of the coil sheath 11a, and the outer surface of the treatment unit support member 14. The electric insulating coating film may be formed on the inner and outer surfaces of the coil sheath 11a without provision of the resin outer cover 11b.

The operation wire 12 is a torque wire which is arranged within the coil sheath 11a to be movable forward and backward, and has a great rotation followability with conductivity. The operation wire 12 may be made of, for example, stainless steel in an entire length or may be formed by connecting a proximal end part made of stainless steel and a distal end part made of nitinol (nickel-titanium alloy) by a stainless pipe.

As shown in Fig. 2, the operation unit 13 is provided with an operation unit main body 13a for operating an opening and closing motion and a rotating motion of the treatment piece, a slider 13b which is slidable with respect to the operation unit main body 13a, a liquid injection part 13c which is connected to a distal end side of the operation unit main body 13a and is provided for injecting the treatment liquid, and a connection cap 13d which is threadably mounted to a distal end side of the liquid injection part 13c.

The operation unit 13 has a liquid injection port (a liquid supply connection port) 13c2 which is disposed in a proximal end part of the coil sheath 11a and injects the treatment liquid into the coil sheath 11a, and is a mechanism for performing a forward and backward moving operation and a rotating operation of the operation wire 12 by an operator.

As shown in Fig. 2, the operation unit main body 13a is provided with an elongated rectangular frame part 13a1 which has a liner guide guiding a linear movement in two rows of side faces and is formed into an elongated rectangular frame shape, a handle part 13a2 which extends to a proximal end side of the elongated rectangular frame part 13a1 and has a circular frame-shaped finger hook hole 13a4 for the operator to hook a finger, and a male type connection part 13a3 which extends to a distal end side of the elongated rectangular frame part 13a1 and is provided for screw attaching to the liquid injection part 13c. The conical male type connection part 13a3 has a through hole which passes through the operation wire 12.

The slider 13b has finger hook holes 13b1 and 13b2 (wing parts) which correspond to holes for the operator to hook the finger, a center part of the slider 13b is slidably fitted to the linear guide of the elongated rectangular frame part 13a1, and is structured such that the slider 13b can slide along the linear guide of the elongated rectangular frame part 13a1 by expanding and narrowing motion of the operator in a state in which the operator hooks a thumb to the finger hook hole 13a4 and hooks an index finger and a middle finger to the finger hook holes 13b1 and 13b2. Thus, the treatment instrument for endoscopes 10 according to the present embodiment can move the operation wire 12 forward and backward within the sheath 11 when the operator holds the operation unit main body 13a of the operation unit 13 and pushes and pulls the finger hooked slider 13b.

The operation wire 12 and the coil sheath 11a are connected so that the proximal ends thereof are integrally rotatable. Therefore, the sheath 11 and the operation wire 12 can be integrally rotated in the distal end side of the sheath 11 by holding the slider 13b, rotating around the elongated rectangular frame part 13a1 and integrally rotating the operation wire 12 and the sheath 11.

In a case where the male type connection part 13a3 is not extended in the elongated rectangular frame part 13a1 so as to integrally extend, but a holding part which separates the male type connection part 13a3 the elongated rectangular frame part 13a1 and does not rotate is provided, and the male type connection part 13a3 is adapted to be relatively rotatably connected to the distal end side of the elongated rectangular frame part 13a1, the operation wire 12 and the coil sheath 11a are relatively rotatable in the distal end side by holding the wing part (the finger hook hole 13b1) of the slider 13b and rotating around the elongated rectangular frame part 13a1.

The liquid injection part 13c has a through hole 13c1 which passes through an inner part in a longitudinal direction, a liquid injection port 13c2 which is connected to an intermediate part of the through hole 13c1, a conical female screw part (no reference symbol) which is formed in a proximal end side of the through hole 13c1 and is threadably mounted to the male type connection part 13a3 of the operation unit main body 13a, and a conical male screw part (no reference symbol) which is formed in a distal end side of the through hole 13c1 and is threadably mounted to the connection cap 13d. The liquid supply means 6 is connected to the liquid injection port 13c2. The liquid supply means 6 is a syringe or a pump, and can send the treatment liquid such as physiological saline solution and hyaluronic acid into the coil sheath 11a.

Further, the operation wire 12 inserted into the sheath 11 is inserted into the connection cap 13d, the through hole 13c1 of the liquid injection part 13c and the through hole of the operation unit main body 13a, and the proximal end of the operation wire 12 is passed to the frame of the elongated rectangular frame part 13a1 and is connected to the slider 13b. A liquid seal ring (not shown) is provided in such a manner as to fill an annular gap between the operation wire 12 and the proximal end side of the through hole of the operation unit main body 13a.

Further, the proximal end side of the sheath 11 is brought into contact with the distal end side of the liquid injection part 13c in a flared manner, and is fixed by the connection cap 13d. The liquid supplied from the liquid supply means 6 is fed to the distal end side of the sheath 11 from the liquid injection part 13c2 through the peripheral space of the operation wire 12 within the sheath 11.

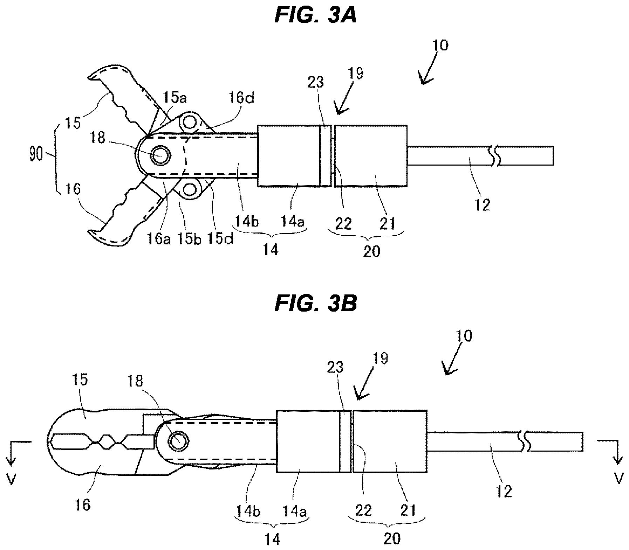

As shown in Figs. 3A and 3B, the treatment unit support member 14 is provided with a tube part 14a which is connected to the distal end part of the coil sheath 11a via a rotatable connection part 19 mentioned later, and has a lid having one closed open face, and a pair of arm parts 14b and 14b which extend to the distal end side from the tube part 14a. In the treatment unit support member 14, an electric insulating coating film is formed on an outer surface thereof, and a hydrophilic coating film is formed thereon in an overlapping manner.

The treatment pieces 15 and 16 and the opening closing links (the third link 83 and the fourth link 84 in Fig. 11) according to the present embodiment are made of the stainless steel or the nitinol (nickel-titanium alloy), and the treatment pieces 15 and 16 construct a pair of cup-shaped biopsy forces. Further, in the present specification, a portion where one overlapped and intersected one ends of a pair of opening and closing links 15b and 16b are connected by the first connection shaft is called as a crossing part. However, in a case where the proximal ends portions of the treatment piece are overlapped and intersected since the treatment piece is constructed by extending from one ends of the opening and closing links 15b and 16b to the distal end side direction, the proximal end portion may be called as a crossing part. The treatment pieces 15 and 16 are structured such that semicircular blade parts come into contact and linear blade parts leading to the semicircular blade parts come into contact in their distal ends, and the electric insulating coating film is provided on the outer surface thereof to be in an electric insulating state each other. Therefore, as shown in Fig. 3B, they are structured such that an electric current flows between the blade parts when the blade parts are closed each other. A pair of treatment pieces 15 and 16 are formed on the hydrophilic coating film so as to overlap the electric insulating coating film.

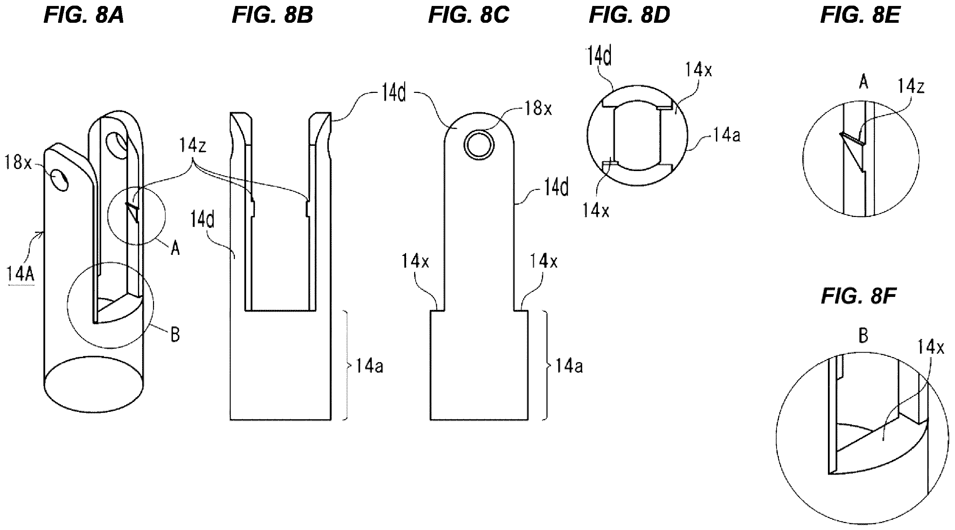

In particular, as shown in Figs. 4A and 4B, the crossing parts 15a and 16a cross and overlap like an X shape between a pair of arm parts 14b and 14b and are provided with shaft holes. A first connection shaft 18 which is supported in both ends to support holes 18x (refer to Fig. 8) of a pair of arm parts 14b and 14b is inserted into the shaft hole, and a pair of treatment pieces 15 and 16 are accordingly supported by a pair of arm parts 14b and 14b.

The treatment unit support member 14 supports the link mechanism constructed by the opening and closing links (the third link 83 and the fourth link 84 in Fig. 11) rotatably connected in one ends thereof around the first connection shaft 88, and a pair of driving links (the first link 81 and the second link 82) connected to the other ends of the pair of opening and closing links by the second connection shaft 86 and the third connection shaft 87, and forms the treatment unit by extending the treatment pieces 15 and 16 from the opening and closing links.

Describing in detail, in the treatment unit support member 14 according to the present embodiment shown in Fig. 4, the opening and closing links 15b and 16b corresponding to the opening and closing links (the third link 83 and the fourth link 84 in Fig. 11) described in the principle drawing are formed thin with a step to overlap, the pin hole is formed, and the opening and closing links 15b and 16b are respectively pin connected (connected by the second connection shaft 86 and the third connection shaft 87 in Fig. 11) to the distal end parts having the pin holes in a pair of driving links 15d and 16d. In the same manner, the distal end portion of the forward and backward movement transmission rod 17 is also formed thin, the pin hole is formed, and the distal end portion is sandwiched in the link proximal end side having the pin hole in a pair of driving links 15d and 16d and is pin connected (connected to the fourth connection shaft 85 in Fig. 11). The electric insulating coating film is formed on the outer surfaces of a pair of driving links 15d and 16d.

The proximal end portion of the forward and backward movement transmission rod 17 and the distal end portion of the operation wire 12 are connected and fixed by fitting the distal end part of the operation wire 12 to a wire accepting hole disposed in an inner portion than the proximal end surface of the forward and backward movement transmission rod 17 and screwing a fastening screw to a female screw disposed in a side surface portion of the wire accepting hole of the forward and backward movement transmission rod 17. The connection and fixing may be achieved by a silver brazing, a soldering or a caulking.

### [Motion of a pair of Treatment Pieces 15 and 16]

In a pair of treatment pieces 15 and 16, a pair of driving links 15d and 16d move so as to enlarge an opening angle when the operation wire 12 is moved to the distal end side. Thus, the opening and closing links 15b and 16b come to an open state [Figs. 3A and 4A] by the application of the forward and backward action force of the operation wire 12, and a pair of treatment pieces 15 and 16 operate so as to set the treatment pieces 15 and 16 to an open state [Fig. 3A and 4A].

Further, in a pair of treatment pieces 15 and 16, a pair of driving links 15d and 16d move so as to reduce the opening angle when the operation wire 12 is moved to the proximal end side. Thus, the opening angle of the opening and closing links 15b and 16b becomes small by the application of the forward and backward action force of the operation wire 12, and a pair of treatment pieces 15 and 16 operate so as to set the treatment pieces 15 and 16 to a closed state.

Therefore, in the treatment unit support member 14 according to the present embodiment, a pair of treatment pieces 15 and 16 open and close on the basis of the operation of the operator for moving the operation wire 12 forward and backward. The treatment unit support member 14 pinches a boundary between a normal part and the treated part (the affected area) of the tissue from the living body by the blade part when closing to take the affected area in the cup, and performs the electrical cauterization associated with a hemostasis action, so that the treatment unit support member 14 can perform treatment such as a resection of the treated part or a sample collection.

### [Characteristic Structure of Treatment Instrument for Endoscopes]

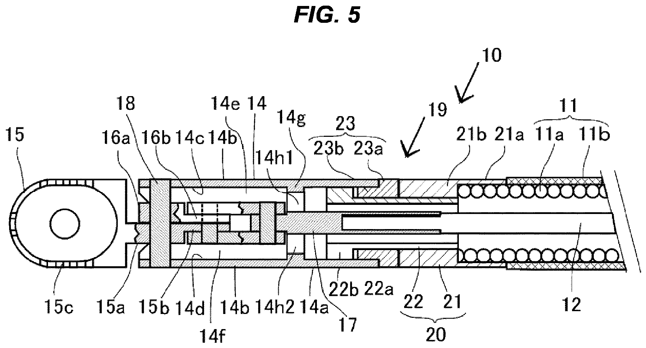

The treatment instrument for endoscopes 10 according to the present embodiment connects the tube part 14a of the treatment unit support member 14 to the coil sheath 11a via the rotatable connection part 19, as shown in Figs. 3A, 3B, 4A, 4B and 5.

The rotatable connection part 19 is provided with the base end side connecting tubular part 20 which includes a tubular coil cap 21 and a connecting tube part 22, and the distal end side connecting tubular part 23 which is formed into a two-stage tubular shape including a large-diameter tube part 23a and a small-diameter tube part 23b and is fixed to the proximal end part of the tube part 14a in the treatment unit support member 14, and the distal end side connecting tubular part 23 is fitted to a connecting small-diameter tube part 22a of the connecting tube part 22 in the base end side connecting tubular part 20 so as to be relatively rotatable and non-detachable.

The base end side connecting tubular part 20 is constructed by two parts including the tubular coil cap 21 and the connecting tube part 22. The tubular coil cap 21 is provided with a thin tube part 21a which is fitted and fixed to the distal end part of the sheath 11, and a thick tube part 21b in which an inner diameter is the same as an inner diameter of the sheath 11 and an inner end is brought into contact with the distal end part of the sheath 11, outer peripheral surfaces of the thin tube part 21a and the thick tube part 21b conform to each other and outer diameters thereof are set to be the same diameter of the tube part 14a of the treatment unit support member 14.

The connecting tube part 22 has the connecting small-diameter tube part 22a and a flange part 22b which is disposed in a distal end side of the connecting small-diameter tube part 22a and slidably fits into the tube part 14a, an approximately half part of the connecting small-diameter tube part 22a is fitted into the thick tube part 21b of the tubular coil cap 21, a fitted end is fixed by welding or brazing, and the remaining approximately half part protrudes out of the thick tube part 21b of the tubular coil cap 21.

The distal end side connecting tubular part 23 is provided with the large-diameter tube part 23a which has the same outer diameter as the diameter of the tube part 14a of the treatment unit support member 14 and is connected and fixed to a direction from the tube part 14a to the sheath 11, and the small-diameter tube part 23b which extends to a front side from the large-diameter tube part 23a and is fitted into the tube part 14a. The large-diameter tube part 23a and the small-diameter tube part 23b have the same inner diameter, and are slidably fitted to the connecting small-diameter tube part 22a of the connecting tube part 22. The large-diameter tube part 23a is connected to the tube part 14a by welding or brazing in their abutting end faces.

Therefore, in the rotatable connection part 19, the operation wire 12 is rotatable with no limitation with respect to the sheath 11. In the rotatable connection part 19, the electric insulating coating film is formed on an outer surface thereof, and the hydrophilic coating film is formed thereon in an overlapping manner.

### [Description of Treatment Liquid Jetting Structure]

The treatment instrument for endoscopes 10 according to the embodiment 1 has the below mentioned structure for energetically jetting the treatment liquid fed within the coil sheath 11a to a forward side of the distal ends of a pair of arm parts 14b and 14b in any of the closed state and the open state of a pair of treatment pieces 15 and 16.

First of all, the treatment unit support member 14 according to the present embodiment constructs a through hole 14h and oblong liquid supply parts 14h1 and 14h2 [Fig. 4D] which are open to an inner concave wall 14g of the tube part 14a in the base end side of the treatment unit support member 14, and treatment liquid supply paths 14e and 14f [Fig. 4C] for circulating the treatment liquid through a gap between curved inner wall surfaces (concave surface parts 14c and 14d) of a pair of circular arm parts 14b extending to the distal end side of the treatment unit support member 14 and an outer surface of the opening and closing link 15b (or 16b) from the crossing part 15a (or 16a) of the driving link 15d (or 16d) and the treatment piece 15 (or 16) arranged between a pair of arm parts 14b.

The treatment liquid supply paths 14e and 14f is formed in such a manner that the treatment liquid is wider than the diameter of the connection shaft (the first connection shaft) connecting the opening and closing links 15b and 16b and the treatment liquid flows straight in both sides of the connection shaft (the first connection shaft).

In order to form the structure, in the treatment unit support member 14 according to the present embodiment, an outer surface of the tube part 14a and an outer surface of each of the concave surface parts 14c and 14d are formed as a cylinder surface in which a center of curvature conforms to an extended center line of the operation wire 12, and an inner surface of each of the concave surface parts 14c and 14d (each of facing surfaces of a pair of arm parts 14b) is formed as a concave tube surface in which a center of curvature conforms to the extended center line of the operation wire 12.

In the present embodiment, there is shown an example in which a shape of the liquid supply part 14h1 open to the inner concave wall 14g is the oval shape. However, the present invention is not limited to this structure, but the liquid supply part may be formed by forming a hole for circulating the treatment liquid separately from the hole passing through the operation wire.

Further, in the treatment unit support member 14 according to the present embodiment, in order to form the structure mentioned above, a gap dimension (a dimension of a gap) between end edges in both sides of the concave surface parts 14c and 14d in the inner surfaces of a pair of arm parts 14b and 14b conforms to a dimension (a width of the link) between the crossing parts 15a and 16a of a pair of opening and closing links 15b and 16b and the outer surfaces of a pair of driving links 15d and 16d, as shown in Figs. 4B and 4C. An outer planar side surface part 15a1 or 16a1 of the crossing part 15a or 16a in the treatment piece 15 or 16 is in slide contact (contact while rubbing) with both side edge parts via the concave surface parts 14c and 14d in the facing surfaces of the facing arm parts 14b.

Further, as shown in Figs. 4B and 4D, the tube part 14a of the treatment unit support member 14 has the inner concave wall 14g, and is provided in the inner concave wall 14g with the oblong through hole 14h which inserts and guides the forward and backward movement transmission rod 17. Further, the through hole 14h is provided with a pair of liquid supply parts 14h1 and 14h2 which flows the treatment liquid supplied through the coil sheath 11a to the center part of the width in each of the concave surface parts 14c and 14d. Thus, the treatment liquid supply paths 14e and 14f are formed by the concave surface part 14c (or 14d) which is formed on the inner surface of each of the arm parts 14b, the opening and closing link 15b (or 16b) from the crossing part 15a (or 16a) in the same side as the one arm part 14b and the side surface parts of a pair of driving links 15d (or 16d). The through hole 14h of this embodiment is open as an oval hole which is long in the facing direction of a pair of arm parts 14b and 14b in such a manner as to have a pair of liquid supply parts 14h1 and 14h2.

As mentioned above, in the treatment unit support member 14 according to the present embodiment, the outer surfaces of a pair of driving links 15d (or 16d) are in slide contact (contact while rubbing) in correspondence to the center part of the width of the concave surface part 14c (or 14d) in the same side arm part 14b. Therefore, the treatment liquid flowing into the center part of the width of the concave surface part 14c (or 14d) from the tube part 14a flows straight due to the action of the water feeding pressure and the surface, and the flow path resistance is increased in the width direction of the concave surface part 14c (or 14d). Thus, an amount of the treatment liquid leaking out of the gap between the arm parts 14b and 14b is reduced, and the treatment liquid jets out of the distal end inner sides of a pair of arm parts 14b and 14b.

### [Description of Jetting Motion of Treatment Liquid]

Subsequently, a description will be given of a state in which the treatment instrument for endoscopes 10 according to the present embodiment jets the treatment liquid fed within the coil sheath 11a to the distal end direction from the distal end of the treatment unit 90 with reference to Figs. 6A, 6B and 6C.

First of all, in the treatment instrument for endoscopes 10 according to the present embodiment, as shown in Fig. 6A, in a case where the treatment liquid is sent in a state in which the treatment pieces 15 and 16 are closed, the treatment liquid flowing out of the distal ends of a pair of treatment liquid supply paths 14e and 14f (the distal ends of a pair of arm parts 14b and 14b) envelops the treatment pieces 15 and 16 in the closed state by the surface tension and forms a one-line shaped liquid flow having a great speed to be energetically jetted forward.

More specifically, the treatment instrument for endoscopes 10 can keep the liquid supply pressure to the crossing parts 15a and 16a by the provision of a pair of treatment liquid supply paths 14e and 14f of the treatment unit support member 14. Further, at the same time, it is possible to accurately jet the treated liquid having a necessarily sufficient liquid supply amount and a sufficient flow velocity to the treated part (the incised affected area and the bleeding area) within the body cavity along a pair of treatment pieces 15 and 16 since the distance from the liquid supply pressure release position to the distal ends of a pair of treatment pieces 15 and 16 is shortened.

Next, in the treatment instrument for endoscopes 10 according to the present embodiment, as shown in Fig. 6B, in a case where the treatment pieces 15 and 16 are inserted into the vicinity of the treated part within the body vagina cavity and the treated liquid is sent in a state in which the treatment pieces 15 and 16 are open to a middle opening degree state, the treated liquid flowing out of the distal ends of a pair of treatment liquid supply paths 14e and 14f flows as two liquid flows along the treatment pieces 15 and 16 by the surface tension, and jets so as to come to one liquid flow in a forward position.

Next, in the treatment instrument for endoscopes 10 according to the present embodiment, as shown in Fig. 6C, in a case where the treatment liquid is sent in a state in which the treatment pieces 15 and 16 are opened at the maximum opening degree, the treated liquid flowing out of the distal ends of a pair of treatment liquid supply paths 14e and 14f is jetted as two liquid flows energetically flowing forward along both side outer surfaces of the treatment pieces 15 and 16 by the surface tension and one liquid flow energetically flowing forward in a fan-shaped open center section of a pair of treatment pieces 15 and 16.

In the treatment instrument for endoscopes 10 according to the present embodiment, in a state in which the treatment pieces 15 and 16 are open at the maximum opening degree, for example, when the treatment pieces 15 and 16 expand the treated part within the body cavity, the treated liquid can be accurately jetted to a whole region of the expanded treated area at a necessarily sufficient liquid supply amount and with a sufficient flow velocity.

Therefore, in the present treatment instrument for endoscopes 10, when the bleeding is removed in a case where the treated area (the cauterization incised area) of the living body has the bleeding, the stagnation or the turbidity, the treatment liquid can flow out as one straight flow from the distal end of the treatment unit support member 14 by coming close to the treated area of the living body in a state in which a pair of treatment pieces 15 and 16 are closed, and sending the physiological saline solution (the treatment liquid) from the liquid supply means 6.

Next, in the present treatment instrument for endoscopes 10, the liquid is divided into two flows from the distal end of the treatment unit support member 14 when a pair of treatment pieces 15 and 16 are opened slowly. Therefore, it is possible to flow and remove the bleeding, the stagnation and the turbidity to both side directions. The present treatment instrument for endoscopes 10 can flow and remove the bleeding, the stagnation and the turbidity more effectively to both side directions by repeating the opening and closing motion of a pair of treatment pieces 15 and 16 and repeating one straight flow and two divided flows of the physiological saline solution flowing out of the distal end of the treatment unit support member 14, as occasion demands, thereby greatly contributing to the treatment.

### [Embodiment 2]

### [Basic Structure of Treatment Instrument for Endoscopes]

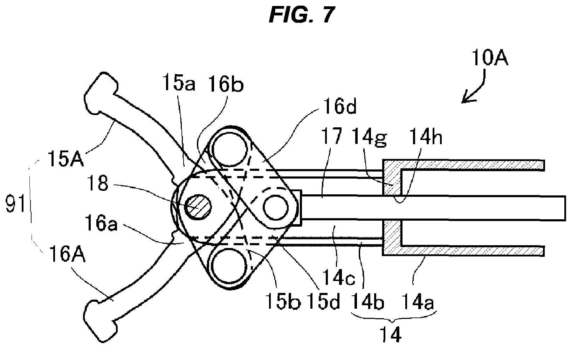

Fig. 7 is a view for describing an outer appearance of a front face of a treatment instrument for endoscopes 10A according to an embodiment 2. The treatment instrument for endoscopes 10A according to the present embodiment is an incision scissors performing an electrical cauterization for an endoscope, and applies a required electric current while pinching a tissue from a living body within a body cavity with a pair of treatment pieces 15A and 16A to incise the tissue from the living body while cauterizing and stopping bleeding.

The treatment instrument for endoscopes 10A is provided with the sheath 11, the operation wire 12, the operation unit 13 and the treatment unit support member 14 which are the same as the embodiment mentioned above, and a treatment unit 91 which is constructed by a pair of treatment pieces 15A and 16A corresponding to a feature of the present embodiment.

Even in the treatment instrument for endoscopes 10A, in the same manner as the treatment instrument for endoscopes 10 according to the embodiment 1, a concave surface part is formed on the inner surfaces of the arm parts 14b and 14b of the treatment unit support member 14, a treatment liquid supply path is accordingly formed between the arm parts 14b and 14b and the treatment pieces 15A and 16A, the inner concave wall 14g having the through hole 14h inserting and guiding the forward and backward movement transmission rod is formed in the tube part 14a, and a pair of liquid supply parts which are the same as a pair of treatment liquid supply paths 14e and 14f shown in Fig. 4C is provided in the through hole 14h.

Further, even in the treatment instrument for endoscopes 10A, the flow of the treatment liquid is the same as Figs. 6A, 6B and 6C.

The treatment instrument for endoscopes 10A according to the present embodiment is provided with the treatment unit 91 constructed by the treatment pieces 15A and 16A corresponding to a pair of scissors pieces which are expanded in distal end portions, and the scissors piece shaped treatment pieces 15A and 16A open and close to pinch the affected area and perform the incision and resection treatment of the affected area by the current application. In the same manner as the embodiment mentioned above, it is possible to accurately jet the treated liquid to the affected area such as the incised and affected area and the bleeding position at a necessarily sufficient liquid supply amount and with a sufficient flow velocity.

### [Embodiment 3]

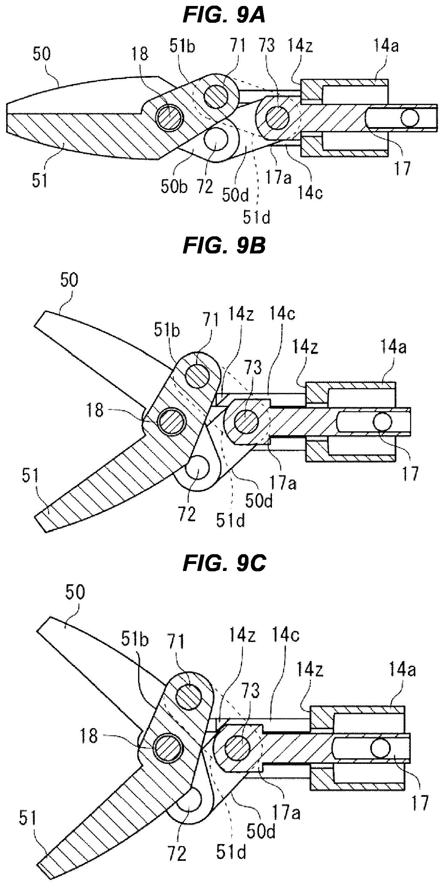

Next, a description will be given of a treatment instrument for endoscopes according to an embodiment 3 of the present invention. A treatment unit support member 14A according to the present embodiment is connected to the distal end part of the coil sheath 11a mentioned above via the rotatable connection part 19, and is provided with the tube part 14a in which an oval liquid supply part passing through the forward and backward movement transmission rod 17 is open to the inner concave wall 14g, a pair of arm parts 14b and 14b in which a cross section thereof extends like a circular arc to the distal end side from the tube part 14a and a support hole 18x rotatably supporting the first connection shaft 18 is open to the distal end part, and the forward and backward movement transmission rod 17 which has a fist-shaped distal end part 17a having a greater diameter than a predetermined shorter diameter of the liquid supply part in the distal end portion passing through the liquid supply part of the tube part 14a, as shown in Figs. 8A and 9A.

The arm part 14b of the treatment unit support member 14A according to the present embodiment is provided, in particular as shown in Figs. 8B and 8E, in an edge end part of an intermediate portion of the arm part with a triangular first stopper 14z for restricting the maximum opening angle of a pair of treatment pieces by the collision of a driving link in a state of opening due to application of the forward and backward moving force from the forward and backward movement transmission rod 17, and is also provided, in particular as shown in Figs. 8D and 8X and Fig. 10, in a circumference portion of an inner concave wall of the tube part 14a with a second stopper 14x for coming into contact with a wide bottom surface (a base end side surface) of the fist-shaped distal end part 17a for restricting the minimum opening angle of a pair of treatment pieces. The first stopper 14z is not limited to the triangular shape, but may be formed into the other shapes such as a circular shape. Further, the stopper for restricting the maximum opening angle is not limited to be arranged in the intermediate portion of the arm part, but may be arranged in the distal end side of the arm part for enlarging the opening angle.

As mentioned above, the stopper 14z restricts the maximum opening angle of a pair of treatment pieces by coming into contact with the driving link to restrict the moving range to the distal end side when the forward and backward movement transmission rod 17 moves to the distal end side. The stopper 14x restricts the minimum closing angle of a pair of treatment pieces by setting a width thereof to the same width of a predetermined shorter diameter of the liquid supply part in such a manner as to come into contact with the wide bottom surface of the fist-shaped distal end part 17a to restrict the moving range to the proximal end side when the forward and backward movement transmission rod 17 moves to the proximal end side. Further, the stopper 14x is not limited to the present example, but may be arranged in such a manner as to come into contact with the narrow portion of the concave surface in the inner side of the arm part.

The treatment instrument for endoscopes according to the third embodiment structured as described above constructs a link mechanism by a pair of driving links 50d and 51d which are rotatably connected in one ends thereof in an overlapping manner by the connection shaft 73 connected to the forward and backward movement transmission rod 17 performing a linear motion, and a pair of opening and closing links 50b and 51b which are rotatably connected in one ends thereof in an overlapping manner to the other ends of the driving links 50d and 51d respectively by the connection shafts 71 and 72, and are rotatably connected in the other ends thereof in an overlapping manner by the first connection shaft 18 position thereof being fixed. A pair of treatment pieces 50 and 51 extending from the opening and closing links 50b and 51b come to a state in which the forward and backward movement transmission rod 17 is a little fed to the distal end side and is accordingly a little opened around the support shaft 18 as shown in Fig. 9B, from a state in which the forward and backward movement transmission rod 17 is pulled to the proximal end side and is accordingly a little closed as shown in Fig. 9A. Further, a pair of treatment pieces 50 and 51 further come to a state in which the forward and backward movement transmission rod 17 is fed to the distal end side and is accordingly a little opened to the maximum opening angle as shown in Fig. 9C. The maximum opening angle is the maximum angle that the driving link 50d comes into contact with the stopper 14z and is restricted to move. As described in the basic structure drawing (Fig. 11) of the link mechanism, the maximum opening angle is restricted by preventing the forward and backward movement transmission rod 17 from moving in the distal end direction by the collision of the driving link 50d with the stopper 14z.

In the present embodiment, there is shown the example in which the stopper 14z comes into contact with the driving link 50d. However, the stopper 14z may be arranged at a position where the opening and closing angle of the opening and closing link is controlled, without being limited to the above example.

The maximum opening angle of the treatment pieces in the treatment instrument for endoscopes is restricted for the following reasons.

According to an opinion from a doctor, when the incision, peeling and collection is performed in a narrow region (a large bowel, a small bowel, a duodenum, a bile duct, a urinary duct, an esophagus and the like) by the use of the high-frequency wave, the treatment unit is positioned near an end surface of the endoscope and the treatment is performed in a state in which a visual field is enlarged. Thus, the treatment instrument for endoscopes has a problem that the visual field of the doctor is obstructed when the maximum opening angle of the treatment pieces is large, a problem that one treatment piece is invisible from the visual field when the treatment pieces are opened largely, a problem that a bowel wall outside the affected area may be injured when the back portions of the treatment pieces are invisible, and a problem that it is dangerous if a position of the other blade is not known when the treatment pieces are opened. Therefore, it is necessary to restrict the maximum opening angle of the treatment instrument for endoscopes and make it apparent for the doctor. In general, about 80 degree to about 90 degree is required as an opening degree that both the blade edges are within the visual field. In a case of a cup, it is not used horizontally like a scissors, an opening angle is required in a narrow region such as a bile duct, a small bowel and a duodenum when it is taken out vertically from the above to the affected arear in a range that a whole thereof is visible by the endoscope and grips the affected area.

Further, the treatment instrument for endoscopes according to the present embodiment is set to the closure minimum angle at which the forward and backward movement transmission rod 17 is pulled to the proximal end side, the wide bottom surface of the fist-shaped distal end part 17a of the forward and backward movement transmission rod 17 comes into contact with the stopper 14x and the movement is restricted.

The minimum closure angle of the treatment pieces in the treatment instrument for endoscopes is restricted for the following reasons.

In the treatment instrument for endoscopes closing the scissors-shaped treatment pieces, in a case where the blade parts are greatly closed and the blade parts are crossed, the lesion may be seized and the blade parts may not be opened. In order to this phenomenon, it is required for the blade parts to be closed without crossing. The blades crossing too much may injure the inner wall of the channel when the endoscope is moved into and out of the channel, and the it is necessary to prevent this phenomenon.

As described above, the treatment instrument for endoscopes according to the present embodiment is provided with the treatment unit support member 14A including the tube part 14a in which the oval liquid supply part is open in the inner concave wall, and a pair of arm parts 14c which extend like a circular arc from the tube part 14a to the distal end side, as shown in Fig. 8. The treatment instrument for endoscopes is structured such that the long tubular forward and backward movement transmission rod including the fist-shaped distal end part and the four-joint link mechanism are embedded in the treatment unit support member 14A in the same manner as the embodiments mentioned above, and the four-link joint mechanism operates to open and close a pair of treatment pieces.

Further, the treatment instrument for endoscopes according to the present embodiment is provided in the intermediate portion of a pair of arm parts 14b with the stopper 14z which restricts the movement of the driving link to the distal end direction, and provided in the tube part 14a with the stopper 14x with which the wide bottom surface of the fist-shaped distal end part 17a of the of the forward and backward movement transmission rod 17 comes into contact. Thus, the maximum and minimum opening and closing angles of the treatment piece 15 and the treatment piece 16 are restricted.

Even in the treatment instrument for endoscopes, the concave surface part is formed on the inner surface of the arm part of the treatment unit support member in the same manner as the treatment instrument for endoscopes according to the embodiment mentioned above. Therefore, the treatment liquid can be jetted along the treatment piece in the same manner as shown in Fig. 6, by forming the treatment liquid supply path in a state in which the circular inner walls of the arm parts and the side surface of the link mechanism are in contact while rubbing, and circulating the treatment liquid passing through the through hole of the tube part to the treatment liquid supply path.

More specifically, the treatment instrument for endoscopes according to the present embodiment is also provided with the treatment unit support member including the tube part which makes the liquid supply part passing the forward and backward movement transmission rod therethrough open to the inner concave wall, and a pair of arm parts which extend like the circular arc from the tube part to the distal end side, and the four-joint link mechanism which is arranged to be sandwiched in a slide contact state between a pair of arm parts of the treatment unit support member, and opens and closes a pair of treatment pieces according to the four-joint link connection, thereby forming the treatment liquid supply path by the gap between the circular inner walls of a pair of arm parts and the side surface of the four-joint link mechanism. Therefore, in the same manner as shown in Fig. 6, the treatment liquid flowed out of the tube part can be jetted along the treatment pieces.

### INDUSTRIAL APPLICABILITY

According to the present invention, the treatment liquid supply path is disposed in the inner surface side of a pair of arm parts in the treatment unit support member, the distance from the release position of the liquid supply pressure to the distal ends of a pair of treatment pieces is shortened as well as the liquid supply pressure is retained to the crossing part of a pair of opening and closing links so as to feed the treatment liquid fed within the sheath from the tube part of the treatment unit support member to the widthwise center part of the treatment liquid supply path disposed in the arm part, and the treatment liquid can be smoothly supplied out of the treatment liquid supply path in the inner surface sides of a pair of arm parts. Therefore, the present invention can provide the treatment instrument for endoscopes which can accurately supply the liquid having the sufficiently great flow velocity along a pair of treatment pieces which can accurately supply the treated liquid having the necessarily sufficient liquid supply amount and the sufficient flow velocity to the treated part within the body cavity along a pair of treatment pieces.

### REFERENCE SIGNS LIST

1 endoscope
2 insertion unit
3 endoscope operation unit
4 treatment instrument introducing unit
5 endoscope channel
6 liquid supply means
10 treatment instrument for endoscopes
11 sheath
11a coil sheath
11b resin outer cover
12 operation wire
13 operation unit
13a operation unit main body
13a1 elongated rectangular frame part
13a2 handle part
13a3 male type connection part
13a4 finger hook hole
13b slider
12a1 finger hook hole
12a2 finger hook hole
13c liquid injection part
13c1 through hole
13c2 liquid injection port (liquid supply connection port)
13d connection cap
14 treatment unit support member
14a tube part
14b arm part
14c, 14d concave surface part
14e, 14f treatment liquid supply path
14g inner concave wall
14h through hole
14h1, 14h2 liquid supply part
15, 16 treatment piece
15a, 16a crossing part
15a1, 16a1 planar side surface part
15b, 16b opening and closing link
15, 16 treatment piece
15d, 16d driving link
17 forward and backward movement transmission rod
18 first connection shaft
19 rotatable connection part
20 base end side connecting tubular part
21 tubular coil cap
21a thin tube part
21b thick tube part
22 connecting tube part
22a connecting small-diameter tube part
22b flange part
23 distal end side connecting tubular part
23a large-diameter tube part
23b small-diameter tube part

## Claims

1. A treatment instrument for endoscopes comprising:
a sheath with flexibility inserted into and removed from an endoscope channel;
an operation wire disposed within the sheath to be able to advance and retreat;
an operation unit disposed in a proximal end part of the sheath, having a liquid injection port injecting a treatment liquid into the sheath and operating the operation wire;
a treatment unit support member having a tube part connected to a distal end part of the sheath and a pair of arm parts extending to a front side from opposite positions to the distal end of the tube part;
a link mechanism constructed by a pair of opening and closing links which are rotatably connected one ends thereof by a first connection shaft in crossing parts intersecting in an overlapping manner, and a pair of driving links which are rotatably connected one ends thereof to the other ends of the pair of opening and closing links respectively by a second connection shaft and a third connection shaft in an overlapping manner, and are rotatably connected the other ends thereof by a fourth connection shaft in an overlapping manner, in which side surfaces of the driving links and/or the opening and closing links overlapping both side edge parts of the pair of arm parts of the treatment unit support member are in contact while rubbing, a position of the first connection shaft being fixed to the arm part;
a pair of treatment pieces adapted to extend in a distal end direction from the one ends of the pair of opening and closing links of the link mechanism, and forming a treatment unit performing treatment of an affected area in a living body by a change between a closed state and an open state of the distal end side; and
a forward and backward movement transmission rod to move the fourth connection shaft forward and backward by a forward and backward action force of the operation wire,
wherein in the treatment unit support member, a concave surface part is formed in an inner surface of the pair of arm parts, the concave surface part being formed into a concave shape in a cross section which is vertical to a longitudinal direction,
wherein an inner concave wall having a through hole inserting and guiding the forward and backward movement transmission rod is formed in the tube part, and the through hole has a liquid supply part allowing the treatment liquid supplied through an inner side of the sheath to flow out to the pair of concave surface parts, and
wherein the arm part has a treatment liquid supply path which jets the treatment liquid flowed out of the pair of concave surface parts, in a gap between the concave surface part formed in the inner surface and a side surface of the link mechanism.

2. The treatment instrument for endoscopes according to claim 1, wherein the treatment liquid supply path is formed in such a manner that the treatment liquid is wider than a diameter of the first connection shaft and the treatment liquid flows straightly in both sides of the first connection shaft.

3. The treatment instrument for endoscopes according to claim 2, wherein the concave surface part is a concave surface which is formed in each of opposite surfaces of the pair of arm parts in such a manner that a center of curvature conforms to an extended center line of the operation wire, and a planar side surface part in an outer side of the crossing part of the opening and closing link is in slide contact with both side edge parts via the concave surface part in the opposite surfaces of the arm part in the same side.

4. The treatment instrument for endoscopes according to any one of claims 1 to 3, wherein the arm part is provided with a stopper which is disposed at a position preventing movement of the driving link and/or the opening and closing link caused by the forward and backward action of the forward and backward movement transmission rod.

5. The treatment instrument for endoscopes according to any one of claims 1 to 3, wherein the forward and backward movement transmission rod is provided with a distal end part having a greater diameter than a shorter diameter of the liquid supply part and formed into a fist shape, in a distal end of the forward and backward movement transmission rod passing through the cylinder tube part.

6. The treatment instrument for endoscopes according to claim 1, wherein the liquid supply part is formed into an oval shape which is longer in the facing direction of the pair of arm parts.

7. The treatment instrument for endoscopes according to claim 2, wherein the liquid supply part is formed into an oval shape which is longer in the facing direction of the pair of arm parts.

8. The treatment instrument for endoscopes according to claim 3, wherein the liquid supply part is formed into an oval shape which is longer in the facing direction of the pair of arm parts.

9. The treatment instrument for endoscopes according to claim 4, wherein the liquid supply part is formed into an oval shape which is longer in the facing direction of the pair of arm parts.

10. The treatment instrument for endoscopes according to claim 5, wherein the liquid supply part is formed into an oval shape which is longer in the facing direction of the pair of arm parts.
